(19) Europäisches Patentamt / European Patent Office / Office européen des brevets

(11) **EP 4 390 840 A1**

(12) **EUROPEAN PATENT APPLICATION**

(43) Date of publication:
**26.06.2024  Bulletin 2024/26**

(21) Application number: **23214522.7**

(22) Date of filing: **06.12.2023**

(51) International Patent Classification (IPC):
**G06T 7/00** *(2017.01)*    *G06V 10/25* *(2022.01)*

(52) Cooperative Patent Classification (CPC):
**G06T 7/0002; G06T 7/0006;** G06T 7/0004;
G06V 10/25

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.12.2022  GB 202219227**

(71) Applicant: **Rolls-Royce plc**
**London N1 9FX (GB)**

(72) Inventor: **Mulloth, Akhil**
**Derby, DE24 8BJ (GB)**

(74) Representative: **Rolls-Royce plc**
**Moor Lane (ML-9)**
**PO Box 31**
**Derby DE24 8BJ (GB)**

(54)    **IMAGE QUALITY QUANTIFICATION AND USES THEREOF**

(57)    A method of quantifying the quality of a scan image of at least a portion of an object against a background, the method comprising: deriving a first measure of contrast and a first measure of sharpness from a first region of the scan image, and a second measure of contrast and a second measure of sharpness from a second region of the scan image; and determining a quality index value indicative of the quality of the scan image based on the first measure of contrast, the first measure of sharpness, the second measure of contrast and the second measure of sharpness.

*500*

S502

S504

*FIG. 5*

EP 4 390 840 A1

**Description**

TECHNICAL FIELD

The present disclosure relates to image quality quantification of an image such as a scan image, and associated methods, apparatuses, computer programs and storage media. This disclosure also relates to uses of such image quality quantification, for example to improve and recognize improved quality of such images.

This disclosure is directed generally to image quality quantification of all types of images, acquired using different types of imaging techniques, and may relate to, for example, X-ray, computerized tomography (CT), magnetic resonance imaging (MRI) or simply scanning.

BACKGROUND

One example type of scanning which generates scan images is CT scanning, in particular X-ray CT scanning.

Three-dimensional (3D) X-ray CT combines multiple X-ray images to recreate a 3D volume. An X-ray source produces polychromatic X-rays that penetrate an object or part thereof, and form an X-ray image on a detector. The object may be rotated through 360 degrees while being imaged multiple times. The X-ray images are combined to produce a 3D representation of the object. Surface determination is then performed to separate the object volume from the background.

CT scanning may be used to inspect non-line-of-sight areas of parts (objects) that are not accessible to alternative inspection techniques such as bore scopes. One such part may be a turbine blade for use in the aerospace industry.

CT scans output CT scan images, which may also be referred to herein as a scan images, (output) images, or simply scans. The scan image may be raw scan data, prior to surface determination. A CT scan image may represent a scan of at least a portion of an object against a background and may be represented as a grey-scale image. A 2DCT scan image may be represented as two-dimensional grid of pixels, and a 3DCT scan image may be represented as a three-dimensional volume of voxels. A 2DCT scan image may be derived from a 3DCT scan image. Each pixel or voxel of the CT scan image may be represented by a grey value in a range of grey values, or grey value in the range of 0 to 65500 Houndsfield units, representing a radiodensity of at least a portion of the object.

In many types of images, contrast and sharpness are known to be important parameters that represent the overall quality of an image. Contrast may be described as the visual ratio of different tones in an image, and sharpness may be described as the clarity or level of detail in an image. Other parameters that represent the overall quality of an image include noise and scatter profiles. A good quality CT scan image should have high contrast, and the edge/s should be sharp (clear, detailed).

Methods of assessing the quality of an image are complex and difficult to administer. It is desirable to provide an improved method of image quality assessment.

STATEMENTS OF INVENTION

In a first arrangement, there is provided a computer-implemented method of quantifying the quality of a scan image of at least a portion of an object against a background. The method comprises deriving a first measure of contrast and a first measure of sharpness from a first region of the scan image, and a second measure of contrast and a second measure of sharpness from a second region of the scan image, and determining a quality index value indicative of the quality of the scan image based on the first measure of contrast, the first measure of sharpness, the second measure of contrast and the second measure of sharpness.

A larger measure of contrast may be indicative of increased contrast. The quality index value may be determined to be proportional to a product of the first and second measures of contrast.

A larger measure of contrast may be indicative of decreased contrast. The quality index value may be determined to be inversely proportional to a product of the first and second measures of contrast.

Each measure of contrast may be dependent on, for the region concerned, a ratio of a value of a pixel or voxel representative of the object and a value of a pixel or voxel representative of the background.

A larger measure of sharpness may be indicative of increased sharpness. The quality index value may be determined to be proportional to a product of the first and second measures of sharpness. A larger measure of sharpness may be indicative of decreased sharpness. The quality index value may be determined to be inversely proportional to a product of the first and second measures of sharpness.

Each measure of sharpness may be dependent on, for the region concerned, the width of an edge transition zone representing an edge where the scan image transitions between the object and the background, and/or a gradient of pixel or voxel values in the edge transition zone.

The first region of the scan image may have relatively high contrast and the second region of the scan image may have relatively low contrast. The relatively high contrast may be higher than a given high contrast threshold. The relatively low contrast may be lower than a given low contrast threshold.

The first region of the scan image may have (substantially) the highest contrast of candidate regions of the scan image. The second region of the scan image may have (substantially) the lowest contrast of the candidate regions of the scan image.

Each of said regions may be a line or cluster of pixels or voxels which includes at least a part of the object and at least a part of the background.

The method may comprise identifying the first and second regions based on user selection and/or based on mathematical analysis of the scan image.

The object may be of a predetermined type, comprising first and second component sections. The first region of the scan image may be selected to align with at least a part of the first section. The second region of the scan image may be selected to align with at least a part of the second section.

In a second arrangement, there is provided a computer-implemented method of assessing the quality of a plurality of scan images, each scan image being of at least a portion of an object against a background. The method comprises quantifying the quality of each scan image using the method of the aforementioned first arrangement, and assessing the quality of the scan images based on their quality index values.

The method may comprise identifying, based on the quality index values, one of the scan images whose quality meets a given quality criterion. The quality criterion may be that the identified scan image has the best quality of the scan images or has a quality above a given threshold value.

The method may comprise, based on the quality index values, identifying a trend or change in scan image quality across the scan images. The plurality of scan images may be a series of scan images and the trend or change is identified across the series of scan images.

The method may comprise obtaining the scan images using an object scanner configured with one or more process variable values, with at least one process variable value different for each scan image. The method may comprise obtaining the scan images using an object scanner configured with the same process variable values for each scan image.

In a third arrangement, there is provided a computer-implemented method of obtaining scan images to meet a given quality criterion, each scan image being of at least a portion of an object against a background. The method comprises obtaining a first scan image using an object scanner configured with one or more process variable values, and quantifying the quality of the first scan image using the method of the aforementioned first arrangement, and, in a searching step, adjusting the configuration of the object scanner by adjusting at least one process variable value, obtaining a subsequent scan image using the object scanner with its adjusted configuration, and quantifying the quality of that subsequent scan image using the method of the aforementioned first arrangement, wherein the method comprises carrying out the searching step at least once or repeatedly at least until the quality index values of the obtained scan images indicate that the given quality criterion is met.

The given quality criterion may be defined such that it is met when: the quality index values of the obtained scan images indicate that at least one of the obtained scan images has a quality above a given threshold value, and/or the quality index values of the obtained scan images indicate that the one of the obtained scan images which has the highest quality has a quality which is a given threshold amount better than that of the scan image with the next best quality, and/or the quality index values of the obtained scan images indicate that the quality of the subsequent scan image of that searching step is better than that of the first scan image or of each other scan image.

The method may comprise adjusting the configuration of the object scanner by referring to a database of image scans and associated process variable values and quality index values, or by referring to a database of process variable values and associated quality index values. The method may comprise adjusting the configuration of the object scanner based on the quality index value of at least one obtained scan image and/or based on the quality index value of each obtained scan image.

Each scan image may be a CT scan image, a 3DCT scan image, or a 2DCT scan image derived from a 3DCT scan image. Each scan image may be obtained using an object scanner. Said object scanner may be a CT scanner. Each scan image may be an image of a cross-section of at least a portion of the object. The object may have at least one internal or exposed cavity. The object may be a turbine blade. Each quality index value may be determined based on a mathematical combination of, or by mathematically combining, the first measure of contrast, the first measure of sharpness, the second measure of contrast and the second measure of sharpness, optionally by multiplication and/or division. Each quality index value may be a single and/or integrated value. Each quality index value may be a simple number and/or a real number.

The method may comprise deriving at least one further measure of contrast and at least one further measure of sharpness from a corresponding further region of the scan image, and determining a quality index value indicative of the quality of the scan image based on the derived measures of contrast and the derived measures of sharpness.

The portion of the object may be a target portion, and the background may comprise another portion of the object distinct from the target portion, or a portion of another object.

In a fourth arrangement, there is provided a quality-quantification apparatus configured to carry out the method of the aforementioned first or second arrangement.

In a fifth arrangement, there is provided a quality-quantification computer program which, when executed on a computer, causes the computer to carry out the method of the aforementioned first or second arrangement.

In a sixth arrangement, there is provided an image scanning system, comprising an object scanner, and a quality-quantification apparatus configured to communicate with the object scanner, wherein the system is configured to carry out the method of the aforementioned first, second or third arrangement.

In a seventh arrangement, there is provided an image scanning computer program which, when executed on a computer of an image scanning system, the system comprising an object scanner and quality-quantification apparatus configured to communicate with the object scanner, causes the system to carry out the method of the aforementioned first, second or third arrangement.

In an eighth arrangement, there is provided a computer-readable medium having the computer program of the aforementioned fifth or seventh arrangement stored thereon.

The inventor of the present invention found that combining contrast and sharpness into a single value provides a powerful and quantitative measure of the quality of an image, in particular a scan image. The single value was extensively used to improve or optimize 3DCT scan quality with excellent results.

LIST OF FIGURES

The present invention is described, by way of example only, with reference to the following drawings, in which:

Figure 1 is a 2D image showing a perspective view of an example object against a background;
Figure 2 is a magnified perspective view of a part of the 2D image of Figure 1, showing how an edge of the object against the background is represented as a group of pixels in the 2D image;
Figure 3 is a plot of the grey values along a line passing through the partial 2D image of Figure 2;
Figure 4 is a plot of the grey values along the line of Figure 2, similar to Figure 3, where a polynomial fit has been applied to the plot of grey values;
Figure 5 is a flow diagram showing the steps of a method embodying the present invention;
Figure 6 is a scan image representative of a first CT scan image of a turbine blade;
Figure 7 presents plots of the grey values, and corresponding gradients of the plots of grey values, along a pair of lines passing through the scan image of Figure 6 corresponding to first and second regions of the turbine blade;
Figure 8 presents two sets of images and plots of first and second CT scans of a turbine blade;
Figure 9 presents two further sets of images and plots of first and second CT scans of a turbine blade;
Figures 10A to 10C are flowcharts of further methods embodying the present invention;
Figure 11 is a schematic diagram of a system comprising quality-quantification apparatus and an object scanner, embodying the present invention; and
Figure 12 is a schematic diagram of a computing device which may serve to implement the quality-quantification apparatus of Figure 11.

DETAILED DESCRIPTION

The present invention relates generally to methods of combining measures of contrast and measures of sharpness into a single value or parameter which indicates the quality of the associated image. This single value or parameter may be referred to as the quality index value or QI (value). The quality index value provides a quantitative measure of the quality of the image. It may also be used to identify drift from nominal image quality due to changes to scanning apparatus machinery or process variables of the scanning apparatus, where the quality index value may decrease signifying a variation or deviation of process variables from a preset value.

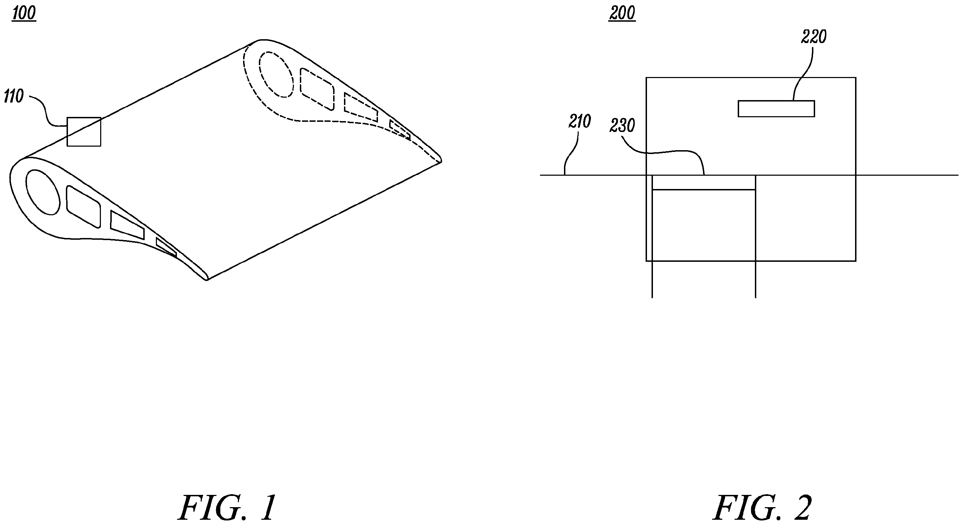

Reference will first be made to Figures 1 to 4.

**Figure 1** is a perspective view of an object against a background, shown as a 2D image. The 2D image is made up of a grid of pixels, as would be understood by those skilled in the art. As shown in Figure 1, box 110 encloses a portion of the lower edge of the object. The image may be understood to be a scan image obtained by an object scanner, such as a CT scanner, in some arrangements. In this example, the object is a turbine blade. For simplicity, in Figure 1 itself the pixels are not explicitly shown having different grey scale values, however based on Figure 2 it can be understood that each pixel may have its own grey scale value.

**Figure 2** is a magnified perspective view of box 110 of Figure 1, showing how an edge of the object against a background is represented as a group of pixels in the 2D image, and how the object would be imaged (and thus showing by shading the grey scale values of the respective pixels).

At this magnified scale, the edge of the object is displayed as a group of pixels with different grey values, within a range of white to black. Certain pixels have intermediary grey values between white and black depending, for example, on how much of the relevant object edge passes through the pixel.

For example, a pixel positioned entirely within the body of the object may be represented as fully or almost white (or light grey as seen on the lower right-hand side of Figure 2). A pixel positioned entirely within the background may be represented as fully or almost black (as seen on the upper left-hand side of Figure 2).

A pixel located on the boundary or border between the object and the background, where the edge of the object passes through part but not all of the pixel, may be represented as a grey value (substantially) between white and black, for example having a value in a mid-range of the full range of values, and may depend on the proportion of the pixel located within the body of the object relative to the proportion of the pixel located within the background. Those skilled in the art will appreciate that graphic images represented as a 2D grid of pixels, or 3D volume of voxels, are typically displayed in this way.

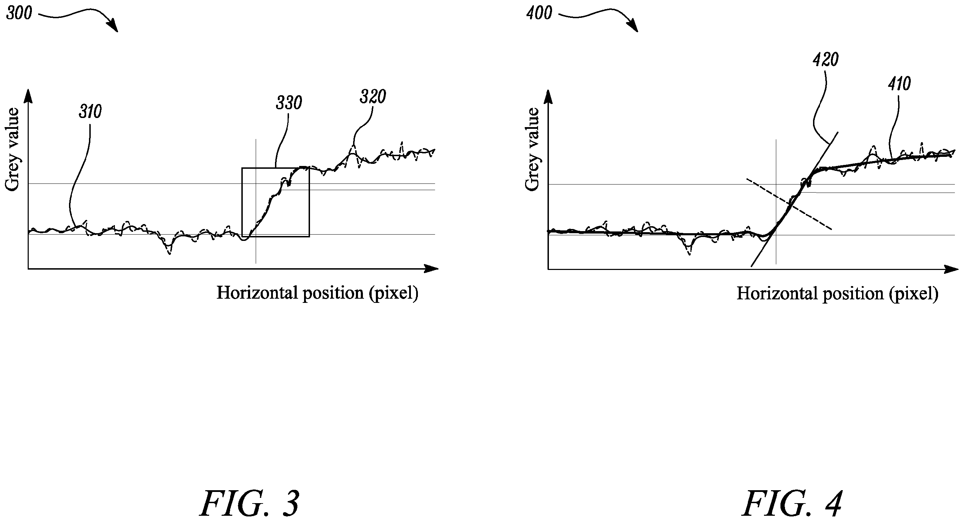

Figure 2 also shows a superimposed line 210 passing through the center of the magnified view of box 110, and a group of pixels 220 which will be discussed later. Figure 2 shows another group of pixels 230 which align with box 330 in Figure 3, and which may be considered a subset of a line of the pixels which may be considered pixels along the line 210 (in this case, the row of pixels immediately below or alongside the line 210).

**Figure 3** is a plot of the grey values of the pixels along line 210 of Figure 2, and thus including the grey values of the pixels 230. The horizontal axis represents the horizontal position of each pixel along the length of the line 210, and the vertical axis represents a measure of the grey value at each pixel. As can be seen, the grey values of the plot are high in regions of the body of the object (representing light grey or white or almost white), and low in regions of the background (representing black or almost black). Moving along line 210 from left to right, the grey values start relatively low where the pixels represent the background (at 310), begin to increase where the pixels represent the edge of the object (within box 330), and finish relatively high where the pixels represent the body of the object (at 320).

**Figure 4** is a plot of the grey values of the pixels along line 210 of Figure 2, similar to Figure 3, where a polynomial fit is applied. A polynomial fit may be applied to remove noise from the original grey value plot. The polynomial fit is represented by a curve 410. Tangent line 420 represents the slope of the curve 410 at a given point or pixel along the curve 410, in particular a point within the portion of pixels representing the object edge and corresponding to a point within box 330.

Against this backdrop, a method of quantifying the quality of a scan image of at least a portion of an object against a background will be described, embodying the present invention.

The following detailed description will consider CT scan images as a running example, however, the methods described herein may be applied to various types of images acquired using different imaging techniques. The methods described herein should not be limited to the types of images described explicitly, and other types of images may be used in the same way as described herein. Colour images may be considered as three grey-scale images stacked on top of one another, where each grey-scale image relates to one of the e.g. RGB colour channels. Thus, the terms "grey-scale" and "grey value" should not be interpreted only to apply to black/white images, and may apply to any colour channel. The following methods may be adapted accordingly.

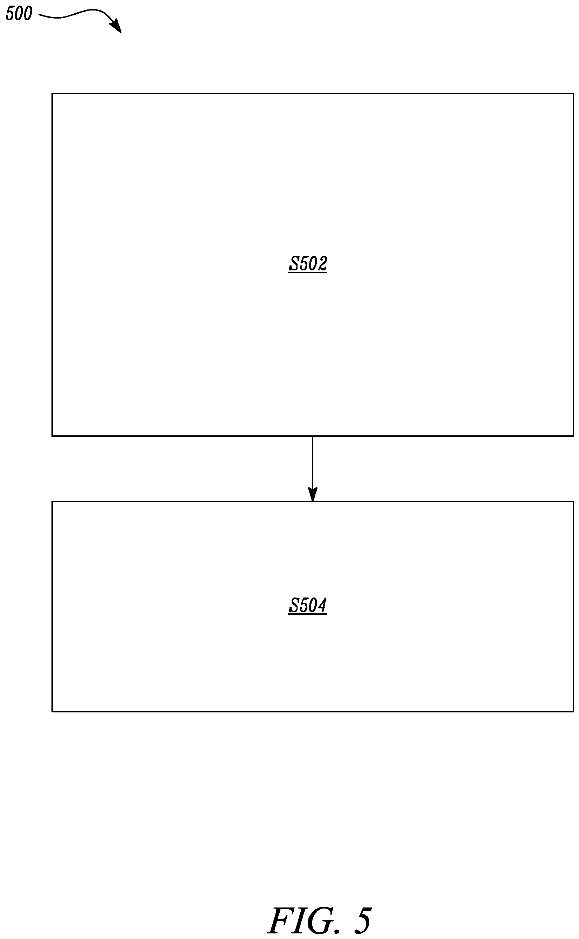

**Figure 5** is a flow diagram showing the steps of a method 500, embodying the present invention.

Method 500 comprises deriving a first measure of contrast and a first measure of sharpness from a first region of the scan image, and a second measure of contrast and a second measure of sharpness from a second region of the scan image (step S502), and determining (e.g. calculating) a quality index value indicative of the quality of the scan image based on the first measure of contrast, the first measure of sharpness, the second measure of contrast and the second measure of sharpness (step S504).

As above, in the running example the scan image is a CT scan image. The scan image may be a 3DCT scan image, or a 2DCT scan image derived from a 3DCT scan image. The scan image may be obtained using a CT scanner, or CT scanning apparatus. The scan image may be an image of a cross-section of at least a portion of the object.

The steps of method 500 will now be considered in turn.

Starting with step S502, the first and second measures of contrast may be referred to simply as measures of contrast. The first and second measures of sharpness may be referred to simply as measures of sharpness.

The first region of the scan image may have relatively high contrast (e.g. contrast higher than a given high contrast threshold), and a second region of the scan image may have relatively low contrast (e.g. contrast lower than a given low contrast threshold), as will be understood from the description of contrast below. The first region of the scan image may have (substantially) the highest contrast and a second region of the scan image may have (substantially) the lowest contrast of candidate regions of the scan image. Candidate regions may refer to a plurality of potential or possible regions of the scan image which may be used as the first and second regions of the scan image.

In general, the object may be any object that has been scanned by an imaging/scanning apparatus. The object may be any 3D object, for example having one or more internal or exposed cavities. However, the object may be of a predetermined type, and with this in mind may be assumed to be a turbine blade, or a portion of a turbine blade, in the running example for ease of understanding. In this context, the object may be considered to comprise first and second component sections (i.e. predetermined component sections where the object is of a predetermined type), and the first region of the scan image may in this case be selected to align with/intersect/correspond to at least a part of the first section and the second region of the scan image may be selected to align with/intersect/ correspond to at least a part of the second section.

Each of the regions may include at least a part of the object and at least a part of the background, and may be a line of pixels. Each of the regions may be a group or cluster of pixels, wherein the group or cluster includes at least a part of the object and at least a part of the background. Where each region is a line of pixels, the line of pixels may be a line of pixels which passes through or across the object. The first and second regions may be identified based on user selection and/or based on mathematical analysis of the scan image, for example to identify regions with given contrast levels such as the highest and lowest contrast, or above/below given high/low contrast thresholds. The first and second regions may be selected by selecting the brightest point and dimmest point of the material (excluding background) as the sampling points for the lines to pass through. These locations and line directions can be selected by calculating eigenvalues and vectors or by experience. Where mathematical analysis is employed to select or position the first and second regions, it may be that those regions do not align with given or expected sections of the object.

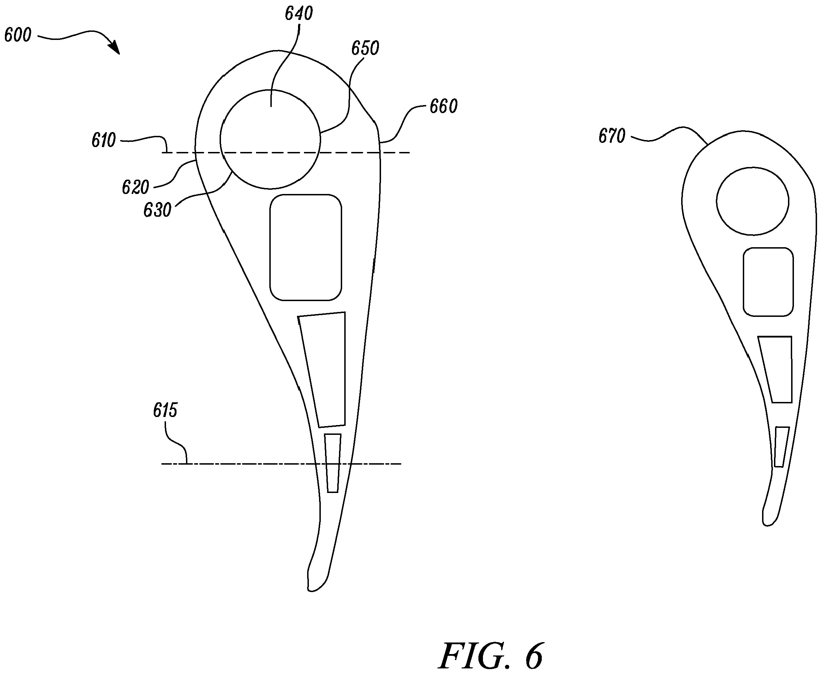

**Figure 6** is a scan image representative of a first CT scan image of a turbine blade, in line with the running example. The turbine blade in Figure 6 is similar in structure to example turbine blade 670, in that it has internal cavities, shown simply so that the internal structure of the scan image can be more readily understood without superimposed reference numerals and dashed lines.

The turbine blade comprises a first component section, being an upper half of the turbine blade in the orientation shown in Figure 6, and a second component section, being a lower half of the turbine blade. In this example, line 610 represents the first region, and line 615 represents the second region.

In the present arrangement, the first region (line 610) of the scan image is selected to align with or be within the first section (the upper half) and the second region (line 615) of the scan image is selected to align with or be within the second section (the lower half). Of course, the first and second regions may refer to any portion of the image within the first and second sections of the object, respectively.

Defining the front to be to the left and the rear to be to the right in Figure 6, merely for convenience, the turbine blade has at the first region (line 610) a front outer edge 620, a front inner edge 630, an internal cavity 640, a rear inner edge 650, and a rear outer edge 660. Line 610 intersects each of the front outer edge 620, front inner edge 630, internal cavity 640, rear inner edge 650 and rear outer edge 660. A front wall of the turbine blade at the first region (line 610) may be considered defined between the front outer edge 620 and the front inner edge 630. Similarly, a rear wall of the turbine blade may be considered defined between the rear inner edge 650 and the rear outer edge 660.

Line 615 intersects either the same or corresponding edges/walls/cavities, as they occur at the second region. It may be that the internal cavity intersected by line 615 is different and separate from the internal cavity 640, or that those cavities are internally linked. It may be that lines 610 and 615 pass through the same edges/walls/cavity in some arrangements, e.g., where the scan image is of only part of a turbine blade.

Regions of voxels will be understood accordingly in 3D. In some arrangements, regions may be planes rather than lines when considering 3D, although lines may also be employed when considering 3D.

Edges and walls/portions of the turbine blade (object) in the vicinity of, or at, these first and second regions are used to quantify or calculate the measures of contrast and measures of sharpness.

Turning first to contrast, each of the first and second regions may have one or more measures of contrast. A measure of contrast of the first and second regions may be a highest measure of contrast in that region, a lowest measure of contrast in that region, an average measure of contrast in that region, or an otherwise representative contrast in that region.

Each measure of contrast may be a contrast ratio, and may be dependent on, for the region concerned, a ratio of a value of a pixel or voxel representative of the object to a value of a pixel or voxel representative of the background. The value of a pixel or voxel representative of the background may be or define a noise floor, and the contrast ratio may be a signal to noise ratio (SNR).

The background simply represents a material, or the absence of material which, upon being imaged, allows the portion of the object (the part of the object being imaged) to be discerned.

The background may represent empty space, or the absence of material. The background may represent a material upon which the object is placed in order to clearly discern the boundary or edge/s of the object, such as the material of a backdrop or a surface upon which the object is placed. The background may be a different material than the object, and may be distinct from the object.

The portion of the object may be a target portion (or a portion of interest), wherein the background comprises another portion of the object distinct from the target portion, or a portion of another object.

For multilateral parts, or complex parts made from combinations of materials, the object and background may be adjacent (or connected) materials, and may be distinguished based on the contrast between (or differences in the values of pixels of) the adjacent materials. The background may be a different portion of the same object formed from a different material, or a different (separate) object formed from a different material.

For example, an object may be formed of two different metal materials. The background may be the first metal material, and the portion of the object being imaged (a target portion or portion of interest) may be formed from the second metal material. In a scan image of at least a portion of an object against a background, the background may represent the first metal material, and the portion of the object may represent the second metal material. Of course, two metal materials are just an example, and the materials may be a metal material and a composite material, or at least three metal materials, or any combination of materials.

As another example, a piece of debris trapped in part of an object during manufacture may be the at least a portion of the object (the target portion), and the material free from debris, i.e., the surrounding material, may form the background. Similar examples include a void or a crack in the material, where the void or crack is the at least a portion of the object, and the material free from voids or cracks, i.e., the surrounding material, is the background.

Each measure of contrast may be the (maximum) difference in grey value (or simply pixel/voxel value) between a first pixel or voxel (representative of the object) of the first region of the scan image, and a second pixel or voxel (representative of the background) of the first region of the scan image, or *vice versa.* That is, the measure of contrast may be the difference in grey value between two different parts of the same region.

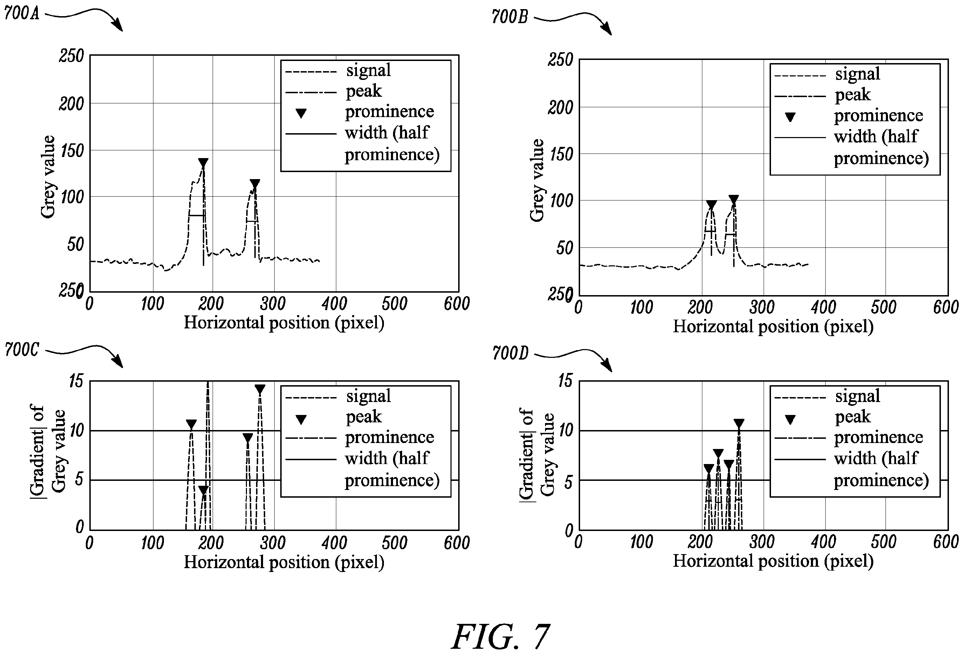

For example, turning back to Figure 2, each measure of contrast may be a difference in grey value between a (substantially) white pixel and a (substantially) black pixel. The contrast ratio may be the difference between the grey value of the lighter material (representing the object) and the darker material (representing the background), or *vice versa.* In the context of Figure 6, note that although the object is shown as being a dark shape on or against a white background, the corresponding plots and scan image portions discussed below in relation to Figures 7 to 9 assume that the object actually appears white or almost white against a black or almost black background. The contrast ratio may therefore be the difference between the grey value of the lighter material (representing the object) and the denser (darker) material (representing the background), or *vice versa.*

**Figure 7** presents plots 700A and 700B of the grey values along lines 610 and 615 of Figure 6, respectively. Plots 700C and 700D show the modulus of the gradients of plots 700A and 700B, respectively.

In detail, plot 700A shows the grey value of each pixel along the line 610, across the width of the scan image of Figure 6. The plot has a relatively low value in the sections of the scan image relating to the background (left of the front outer edge 620, inside the internal cavity 640, and right of the rear outer edge 660) and has a relatively high value in the sections of the scan image relating to the walls of the turbine blade (between the front outer edge 620 and front inner edge 630, and between the rear inner edge 650 and the rear outer edge 660). These relatively high values appear in the grey value plot as pulses, bordered by rising and falling edges corresponding to the edges of the turbine blade (i.e., the front outer edge 620, the front inner edge 630, the rear inner edge 650 and the rear outer edge 660).

For example, a measure of contrast of the first region may be the difference (or ratio) in grey value between the peak of the first (left-most or front-most) pulse representing the front wall of the turbine blade and the noise floor representing the background, or the peak of the second (right-most or rear-most) pulse representing the rear wall of the turbine blade and the noise floor representing the background, or an average of these differences/ratios. In one arrangement a measure of contrast of the first region may be the ratio in grey value between the peak of the second pulse representing the rear wall of the turbine blade and the noise floor representing the background, given that this represents the "worst" (lowest) contrast value between the two walls of the turbine blade at the first region.

Plot 700B shows the grey value of each pixel along the line 615, across the width of the scan image of Figure 6, and a measure of contrast of the second region may similarly be determined. In one arrangement a measure of contrast of the second region may be the ratio in grey value between the peak of the first pulse representing the front wall of the turbine blade and the noise floor representing the background, given that this again represents the "worst" (lowest) contrast value between the two walls of the turbine blade at the second region.

The peaks of the pulses and values of the noise floor representing the background may be identified based on user selection or based on mathematical analysis of the scan image. The edges of the turbine blade may be identified based on analysis of the grey value, or analysis of the gradient (or the modulus of the gradient) of the grey values or slope of a polynomial fit of the grey values.

Turning second to sharpness, each measure of sharpness may be a width of an edge transition zone. Each measure of sharpness may be dependent on, for the region concerned, the width of an edge transition zone representing an edge of the turbine blade in the 2D image being a boundary or border between the object and the background. Each measure of sharpness may be dependent on a gradient or slope of a polynomial fit of the grey values in the edge transition zone, as explained in relation to Figure 4.

The edge transition zone may refer to a region (or zone) of the scan image representing an edge of the object (e.g. turbine blade). As before, at magnified scale, the edge of the object is represented as a group of pixels of different grey values. Referring to Figure 2, the width of the edge transition zone may be the number of pixels required to transition from a pixel having a value representing the background (i.e., black) to a pixel having a value representing the object (i.e., light grey or white). The edge transition width as a number of voxels may be understood similarly in 3D.

In Figure 2, along line 210, it can be seen that a black pixel adjacent the edge is separated from a light grey or white pixel by five pixels having intermediary grey values (box 220 for example shows another five pixels having intermediary grey values). The width of the edge transition zone is thus seven pixels (shown in box 230).

The first measure of sharpness may be a gradient or slope of a grey-value intensity plot or polynomial fit curve of the grey values in a grey value plot, as shown in Figures 3 and 4, and may be e.g., the gradient of tangent line 420. The gradient may be calculated based on any point along the intensity plot or polynomial fit curve, and the measure of sharpness may be the maximum value of the gradient.

It will be understood by the skilled person that there exists a relationship between the width of the edge transition zone and the gradient of the grey value intensity plot, and that one may be calculated based on the other.

The first measure of sharpness may also be referred to as a measure of blurriness, or a measure of the inverse of sharpness. A measure of the inverse of sharpness may be understood as 1 divided by the measure of sharpness.

Returning to the running example of Figures 6 and 7, it is recalled that plots 700C and 700D show the modulus of the gradients of plots 700A and 700B, respectively.

In detail, plot 700C shows the gradient of the grey values of pixels along the line 610, across the width of the scan image of Figure 6. The plot 700C therefore has four distinct spikes corresponding to the four edges of plot 700A, i.e. the front outer edge 620, front inner edge 630, rear inner edge 650 and rear outer edge 660. One way to take a measure of sharpness of the four edges of the first region 610 would be to measure the peak values of the corresponding spikes of plot 700C. Another way to take a measure of sharpness of the four edges of the first region 610 would be to measure the widths of the corresponding spikes of plot 700C, for example the widths at half of the peak values of the corresponding spikes of plot 700C, or as another example the widths at the base of the spikes (i.e., the maximum width of the corresponding spikes of plot 700C).

For example, a measure of sharpness of the first region may be width at half the peak value of the first (left-most or front-most) spike representing the front outer edge 620, of the second spike representing the front inner edge 630, of the third spike representing the rear inner edge 650, of the fourth (right-most or rear-most) spike representing the rear outer edge 660, or an average of these widths. In one arrangement a measure of sharpness of the first region may be the width at half the peak value of the spike which has the widest or largest such width, given that this represents the "worst" sharpness value at the first region.

Plot 700D shows the gradient of the grey values of pixels along the line 615, across the width of the scan image of Figure 6, and a measure of sharpness of the second region may similarly be determined. In one arrangement a measure of sharpness of the second region may be the width at half the peak value of the spike which has the widest or largest such width, given that this represents the "worst" sharpness value at the second region.

The widths or slopes may be user measured or calculated based on mathematical analysis of the scan image. The edges and edge transition zones of the turbine blade may be identified based on analysis of the grey value, or analysis of the gradient of the grey values or slope of a polynomial fit of the grey values.

Method 500 comprises determining (e.g. calculating) a quality index value indicative of the quality of the scan image based on the first measure of contrast, the first measure of sharpness, the second measure of contrast and the second measure of sharpness, at step 504.

The quality index value may take the form of a product of the first measure of contrast, the first measure of sharpness, the second measure of contrast and the second measure of sharpness, particularly where a larger measure of contrast is indicative of increased contrast and a larger measure of sharpness is indicative of increased sharpness. The quality index value may take the form of a quotient of the first measure of contrast and the first measure of sharpness, multiplied by a quotient of the second measure of contrast and the second measure of sharpness, particularly where a larger measure of contrast is indicative of increased contrast and a larger measure of sharpness is indicative of decreased sharpness.

Thus, it will be apparent that a larger measure of contrast may be indicative of increased contrast, and in this case the quality index value may be calculated (or determined, e.g. based on a look-up table) to be proportional to a product of the first and second measures of contrast. A larger measure of contrast may be indicative of decreased contrast, and in this case the quality index value may be calculated (or determined, e.g. based on a look-up table) to be inversely proportional to a product of the first and second measures of contrast.

Similarly, a larger measure of sharpness may be indicative of increased sharpness, and in this case the quality index value may be calculated/determined to be proportional to a product of the first and second measures of sharpness. A larger measure of sharpness may be indicative of decreased sharpness, and in this case the quality index value may be calculated/determined to be inversely proportional to a product of the first and second measures of sharpness.

As above, the quality index value may take the form as generally given below, where a larger measure of contrast is indicative of increased contrast and a larger measure of sharpness is indicative of decreased sharpness:

$$Quality\ Index\ Value\ or\ QI = \frac{First\ measure\ of\ contrast}{First\ measure\ of\ sharpness} \times \frac{Second\ measure\ of\ contrast}{Second\ measure\ of\ sharpness}$$

The above formula may also be represented as:

$$Quality\ Index\ Value\ or\ QI$$
$$= First\ measure\ of\ contrast \times \frac{1}{First\ measure\ of\ sharpness}$$
$$\times\ \ Second\ measure\ of\ contrast \times \frac{1}{Second\ measure\ of\ sharpness}$$

and will be understood according to the above description of a measure of sharpness (e.g. spike width) being an inverse of a measure of sharpness.

Each measure of contrast and each measure of sharpness upon which the quality index value is based may be provided with an associated weight w1 to w4, as generally given below:

$$Quality\ Index\ Value\ or\ QI = \frac{w1 * First\ measure\ of\ contrast}{w3 * First\ measure\ of\ sharpness} \times \frac{w2 * Second\ measure\ of\ contrast}{w4 * Second\ measure\ of\ sharpness}$$

It should be understood that the associated weights may be implemented in any of the above definitions of the quality index value. Weights may be chosen depending on a specific use case, or when performing imaging on a particular object. Weights may be chosen by an operator of the imager, or may be determined by previous experimentation. The weights may serve to favour or emphasise measures of contrast versus measures of sharpness or *vice versa.*

While method 500 has been described using first and second measures of contrast and sharpness, it shall be understood that a single measure of contrast and a single measure of sharpness may be used to determine a quality index value based on a single region of the image. These measures may be used to determine a quality index value in a manner similar to that described above. While two regions are described in the above example, the quality index parameter may be calculated as a product or quotient involving the single measure of contrast and the single measure of sharpness only.

That is, method 500 may be a computer-implemented method of quantifying the quality of a scan image of at least a portion of an object against a background, the method comprising deriving a first measure of contrast and a first measure of sharpness from a first region of the scan image, and determining a quality index value indicative of the quality of the scan image based on the first measure of contrast and the first measure of sharpness.

Similarly, at least a further measure of contrast (i.e., three or more measures of contrast) and at least a further measure of sharpness (i.e., three of more measures of sharpness) may be determined from a corresponding further region of the scan image (from a third region for the third measure, from a fourth region for a fourth measure etc.). The measures may be combined in a manner similar to that described above. It should be understood that n measures of contrast and n measures of sharpness based on n respective regions of the scan image may be used in order to determine a quality index value, where n is a positive integer greater than or equal to 1.

That is, method 500 may be a computer-implemented method of quantifying the quality of a scan image of at least a portion of an object against a background, the method comprising deriving at least one further measure of contrast and at least one further measure of sharpness from a corresponding further region of the scan image, and determining a quality index value indicative of the quality of the scan image based on the derived measures of contrast and the derived measures of sharpness.

Although it is apparent from the above equations that QI values may be obtained by calculation, the skilled person will recognize that a look-up table of QI values, accessed by the different measures, may be employed to achieve the same effect. The present disclosure will be understood accordingly. The terms determination and calculation will thus be understood to encompass any way of obtaining the QI values from the different measures.

It is recalled that the scan image of Figure 6 is representative of a first CT scan image of the turbine blade. A quality index (QI) value of the first scan image determined according to the above-described formula may thus be considered a first QI value.

It will be understood that a second CT scan image of the turbine blade could be obtained with the CT scanner (object scanner) configured differently, i.e. with at least one process variable value adjusted compared to the configuration in which the first CT scan image was obtained (where process variable values configure the scan process). The second CT scan image would then be different from the first CT scan image of Figure 6. Also, if the quality of the second CT scan image is assessed in the same way as the first CT scan image, plots corresponding to those of Figure 7 would be obtained and a corresponding second QI value determined/calculated, different from the first QI value.

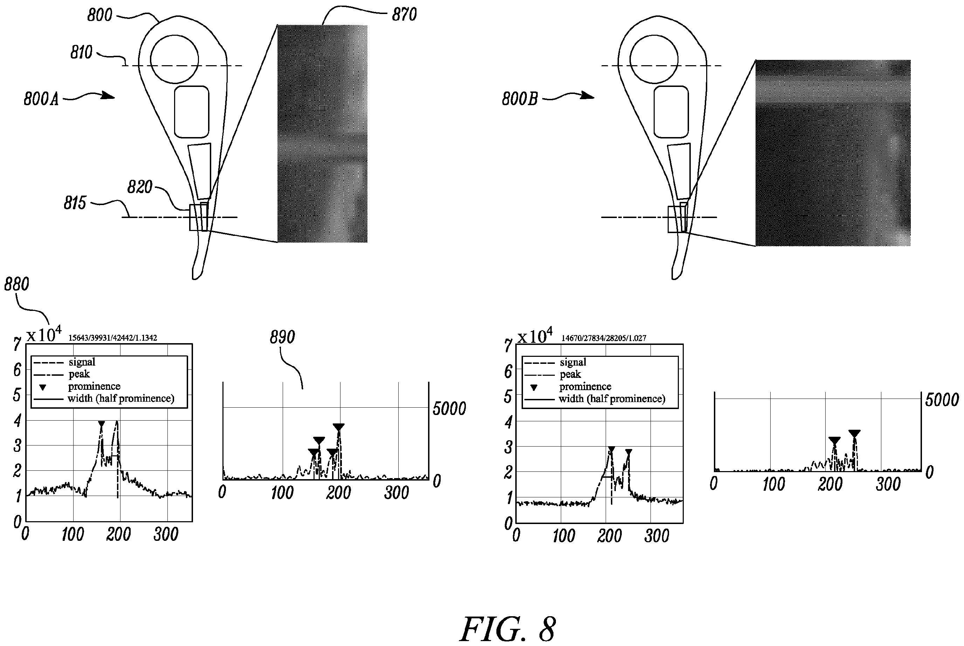

An example in which two CT scans of the turbine blade are compared, and upon which an assessment as to the quality of the CT scans may be made, will be described in relation to Figure 8.

**Figure 8** presents two sets of images and plots of first and second CT scans of a turbine blade. The first set 800A corresponds to the first CT scan, and the second set 800B corresponds to the second CT scan.

Set 800A comprises an image of a turbine blade 800. The turbine blade 800 is similar to turbine blade 600 of Figure 6. Lines 810 and 815 are shown, representing first and second regions of the turbine blade (and which correspond to lines 610 and 615 of Figure 6). For simplicity, only the second region (i.e., line 815) will be considered.

A magnified portion of a first CT scan image 870 of the turbine blade 800 is shown, corresponding to a magnified view 870 of the first CT scan at the front outer edge 820. Moving from left to right across the magnified view 870, the image transitions from the background (represented as black) to the body of the turbine blade (represented as light grey or white). The line across the width of the magnified portion represents line 815.

Also shown is a grey value plot 880 which plots the grey value of the pixels along the entire length of line 815, and a plot 890 of the gradient of the grey values of pixels along the entire length of line 815. Plots 880 and 890 may be likened to plots 700B and 700D respectively.

A first QI value based on the results of the first CT scan was calculated to be 168, using measures of contrast and measures of sharpness from the first CT scan image, and represents a relatively high quality CT scan image, where the object body is distinct from the background.

Set 800B comprises images and plots of a second CT scan of the turbine blade 800, and represent the same things as those in their equivalent images and plots in collection 800A. For simplicity, duplicate reference numerals have been omitted.

A second QI value based on the results of the second CT scan was calculated to be 48, and represents a relatively low quality scan image, where the object body is not as distinct from the background as in set 800A, and where the front outer edge 820 is blurry.

Comparing the grey value plots of the sets, it is apparent that the contrast between the background and the body of the object is greater in set 800A, evidenced by the difference between the minimum and maximum grey value. Comparing the plots of the gradient of the grey values of the sets, it is also apparent that the sharpness of the scan image of set 800A is greater than that of set 800B, evidenced by the higher peaks in set 800A.

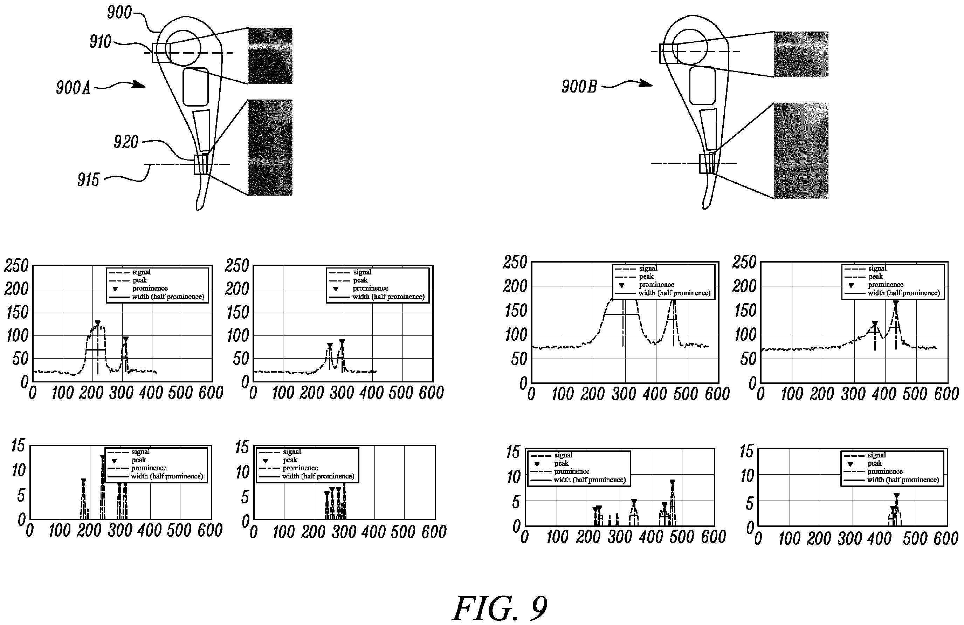

**Figure 9** presents two further sets of images and plots of first and second CT scans of a turbine blade 900. The first set 900A corresponds to the first CT scan, and the second set 900B corresponds to the second CT scan.

The images and plots of Figure 9 are largely comparable to the images and plots of Figure 8, and so repeat description of what the images show and what the plots represent shall be omitted.

Notably however, Figure 9 shows two magnified views from each CT scan image of turbine blade 900, representing magnified views at the front outer edge 920 of both the first and second regions of the turbine blade. The lines across the widths of the first and second magnified views represent lines 910 and 915 respectively. Grey value plots and plots of the gradient of the grey values of pixels are shown for both lines 910 and 915, representing first and second regions of the scan image. In both sets 900A and 900B, plots on the left correspond to line 910, and plots on the right correspond to line 915.

Comparing the grey value plots of the sets, the contrast in set 900A is less than the contrast in set 900B, evidenced by the difference between the minimum and maximum value of the grey value plot. This can be seen in the magnified views, where set 900B shows a relatively brighter CT scan image, allowing greater contrast between the background and object body.

Comparing the plot of the gradient of the grey values of the sets, it appears that the sharpness of the scan image of set 900A is greater than that of set 900B, evidenced by the higher peaks on these plots in set 900A compared to those in set 900B.

A first QI value based on the results of the first CT scan was calculated to be 48, and represents a relatively high quality CT scan image, where the object body is distinct from the background and the boundary of the front outer edge 920 is sharp. A second QI value based on the results of the second CT scan was calculated to be 5, and represents a relatively low quality scan image, where the object body is not as distinct from the background as in set 900A, and where the front outer edge 920 is blurry.

If the second QI value is larger than the first QI value it may be concluded that the change in process variable value(s) has produced a higher or better quality scan image. Conversely, if the second QI value is smaller than the first QI value (as in the examples above) it may be concluded that the change in process variable value(s) has produced a lower quality scan image. That is, in the running example it is assumed that when the quality of the image increases, the quality index value increases, and *vice versa.* In another example, however, when the quality of the image decreases, the quality index value increases, and *vice versa.*

A better quality image may strike a balance between measures of contrast and sharpness at the two regions by effectively averaging measures of contrast and sharpness in these two regions. For example, where the first region has a relatively high measure of contrast and a relatively high measure of sharpness, and the second region has a relatively low measure of contrast and a relatively low measure of sharpness, a better quality image may be an image where the measure of contrast and the measure of sharpness of both regions are measures in between the relatively low measure and the relatively high measure.

The quality quantification techniques disclosed herein may be used in various ways. For example, the QI values may be used to assess quality, or relative quality, amongst a plurality of scan images, or to obtain a scan image with improved quality or with quality meeting a given (e.g. predetermined) criterion. The quality quantification techniques disclosed herein represent scan image quality as a single value (the quality index, QI, value), facilitating such uses. For example, QI values allow for the process variables of the object scanner to be tuned to improve or optimise the scan quality, based on a (single, integrated) quantitative assessment of image quality.

In each of the following example methods it is assumed that each scan image is of at least a portion of an object against a background, as before. Parts of the methods relate to steps previously described above, and so repeat description will be omitted. CT scan images may be considered, as in the running example, however it is recalled that the methods described herein may be applied to various types of images acquired using different imaging techniques. The methods described herein should not be limited to the types of images described explicitly. For example, while CT scan images, MRI images and X-ray images are explicitly envisaged, other types of image may be used in the same way as described herein.

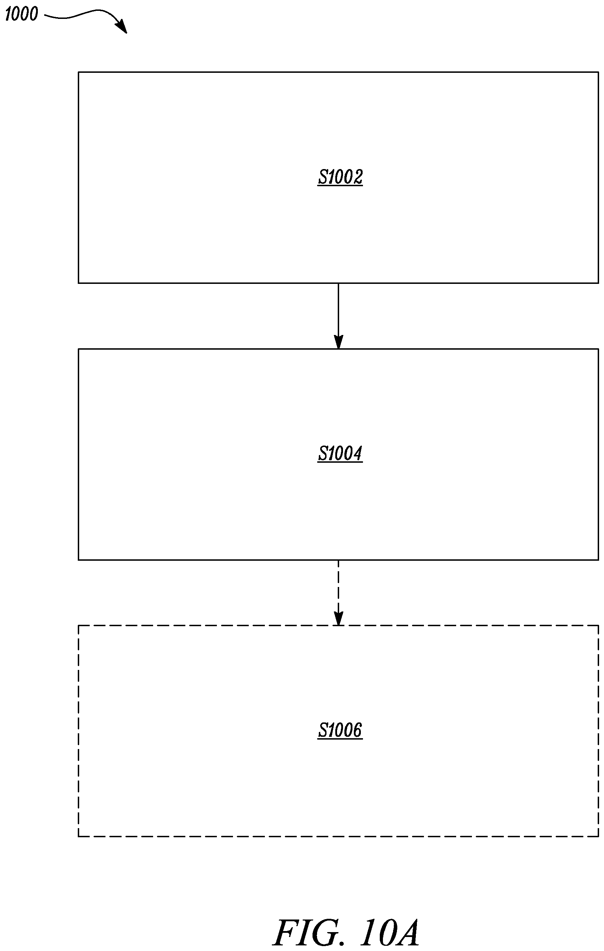

**Figure 10A** is a flowchart of a method 1000 of assessing the quality of a plurality of scan images, comprising steps S1002, S1004 and optionally S1006. In step S1002, the quality of each scan image is quantified using the techniques described above, for example method 500, and therefore generating a quality index value (QI value) per scan image. In step S1004, the quality of the scan images is then assessed based on their quality index values.

Method 1000 may comprise identifying, in optional step S1006, based on the quality index values, one of the scan images whose quality meets a given quality criterion. The quality criterion may be that the identified scan image has the best quality of the scan images or has a quality above a given threshold value (e.g. a QI value above a given threshold value). Method 1000 may comprise obtaining the scan images using an object scanner configured with one or more process variable values, with at least one process variable value different for each scan image.

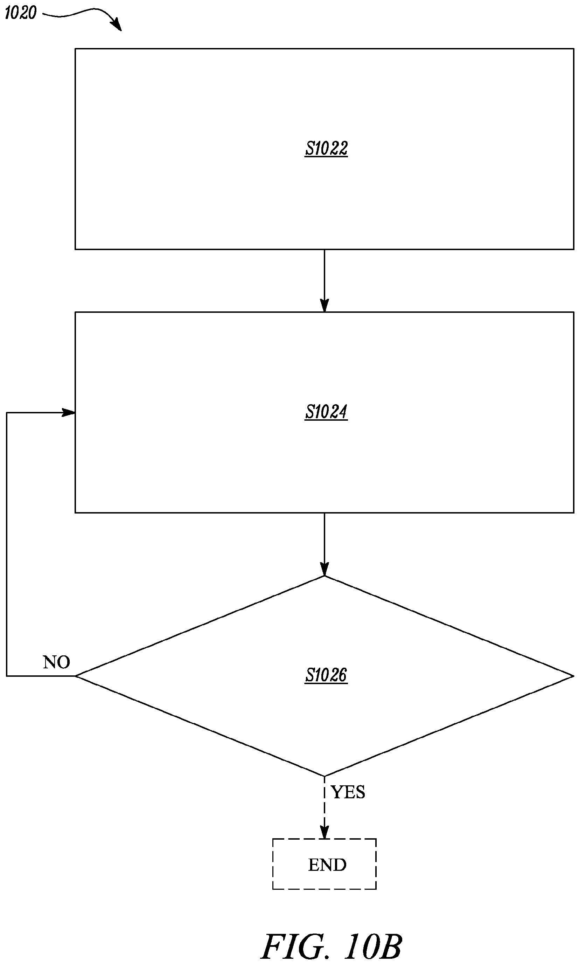

**Figure 10B** is a flowchart of a method 1020 of obtaining scan images to meet a given quality criterion, comprising steps S1022, S1024 and S1026. In step S1022, a first scan image is obtained using an object scanner configured with one or more process variable values, and the quality of the first scan image is quantified using the techniques described above, for example method 500, generating a QI value. In step S1024, a searching step, the configuration of the object scanner is adjusted by adjusting at least one process variable value, a subsequent scan image is obtained using the object scanner with its adjusted configuration, and the quality of that subsequent scan image is quantified using the techniques described above, for example method 500, generating a QI value. In step S1026, it is determined if the given quality criterion is met. If not (S1026, NO), the method returns to step S1024. As such, method 1020 comprises carrying out the searching step at least once or repeatedly at least until the quality index values of the obtained scan images indicate that the given quality criterion is met. Method 1020 may end when the given quality criteria is met (S1026, YES).

The given quality criterion may be defined such that it is met when the quality index values of the obtained scan images indicate that at least one of the obtained scan images has a quality above a given threshold value. As another example, the given quality criterion may be defined such that it is met when the quality index values of the obtained scan images indicate that the one of the obtained scan images which has the highest quality has a quality which is a given threshold amount better than that of the scan image with the next best quality. As another example, the given quality criterion may be defined such that it is met when the quality index values of the obtained scan images indicate that the quality of the subsequent scan image of that searching step is better than that of the first scan image or of each other scan image. These are of course just examples. As above, representing scan image quality as a single value (the quality index value) allows the process variables of the object scanner to be tuned to improve or optimise scan quality, based on a (single, integrated) quantitative assessment of image quality.

The configuration of the object scanner may be adjusted by referring to a database of image scans and associated process variable values and quality index values, or by referring to a database of process variable values and associated quality index values. The database may be based on existing or previously performed image scans of the same or of different objects. The configuration of the object scanner may be adjusted based on the quality index value of at least one obtained scan image and/or based on the quality index value of each obtained scan image. Thus, one or more previous QI values may be used to inform how the configuration of the object scanner is adjusted.

For example, the database may be searched for a quality index value that is higher (or lower) than an existing quality index value, and the associated process variable values of the higher (or lower) quality index value are used as basis to adjust the configuration of the object scanner. The object scanner may be configured to use the same associated process variable values of the higher (or lower) quality index value. The object scanner may be configured to use process variable values similar to, or more similar to, those of the higher (or lower) quality index value, or process variable values between existing process variable values and the associated process variable values.

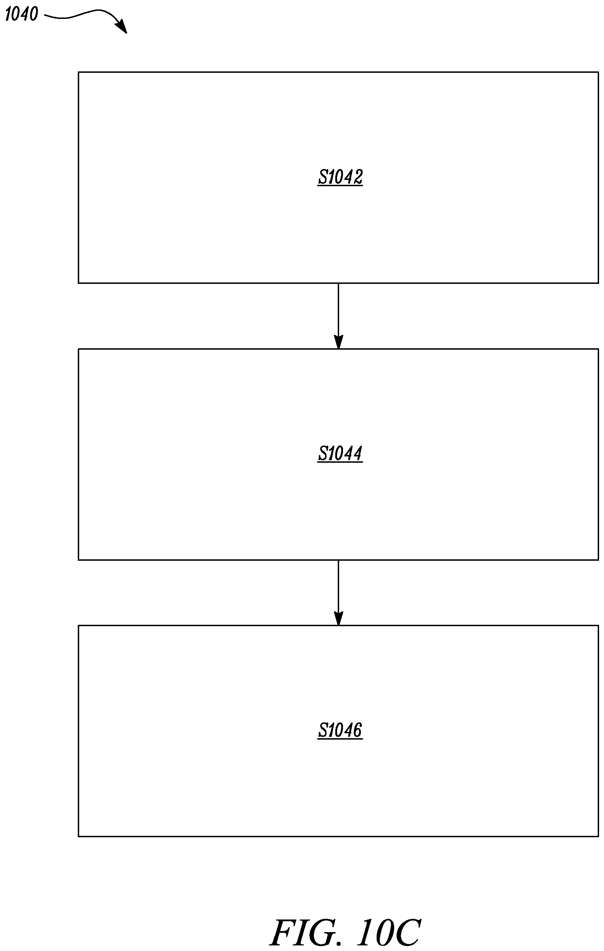

**Figure 10C** is a flowchart of a method 1040 of tracking scan image quality, comprising steps S1042, S1044 and S1046. In step S1042, a series of scan images is obtained using an object scanner. In step S1044, the quality of each scan image is quantified using the techniques described above, for example method 500, and therefore generating a quality index value (QI value) per scan image. In step S1046, based on the quality index values, a trend or change in scan image quality is identified across the series of scan images. In this way, as mentioned earlier, it may be possible to quantitatively identify drift from nominal image quality due to changes to the object scanner (e.g. ageing, or worsening condition) or its process variable values (e.g. mistaken reconfiguration of the scanner). For example, the quality index value may decrease signifying a variation or deviation of process variable values from a preset value.

Thus, a deviation in process variable values of the object scanner that leads to a change in scan quality can be monitored by monitoring the quality index value. That is, the process variable values may initially be set to an ideal or default value. By repeatedly obtaining subsequent scan images (over time) without adjusting the process variable values to improve the scan quality, the quality index value of subsequent images can be compared. If the quality index value of the subsequent images begins/starts to deteriorate (if the quality index value of a subsequent image is outside an established threshold or error range of the ideal or default value), the method may identify this as a deviation of the process variable values from their ideal or default values, and triggers an alert to notify a user that the process variable values may have changed, or that maintenance is needed. Such a method may prevent acquiring scan images whilst the scanner is in an incorrect configuration.

Where the object scanner is for example a CT scanner, as in the running example, it may be configured with a number of different process variables. Example process variables include an operating or scanning voltage, current, or beam energy. The process variables may also include an orientation of the object relative to the scanner, magnification of the object, or a number of slices, projections or scans taken using the CT scanner, the slice thickness, scan interval, scan time, rotation time, helical pitch, beam collimation etc. The process variables may be referred to as key process variables (KPVs).

Returning to method 1020, and considering the quality criterion to require an improvement or optimization or other comparison in scan quality, by using the process variable values of an associated scan image that has a higher quality index value, the average measures of contrast and sharpness across the image may be improved overall. This may involve a reduction in the measure of contrast or sharpness in one region of the image in order to improve the measure of contrast or sharpness in another region, but where the image as a whole has an improved quality index value (a quantitative measure of a combination of contrast and sharpness). Further, selection of the lowest measures of contrast in each of the first and second regions of the scan image while initially quantifying the quality of the scan image may allow for the greatest improvement in image quality, since use of the lowest measure of contrast effectively provides a worst-case scenario quality index value of the image which is to be improved.

Method 1020 may comprise carrying out the searching step a plurality of times to obtain a succession of different subsequent scan images obtained with the object scanner having respective different configurations. That is, the method may iterate, or repeat, the searching step (adjusting the object scanner, obtaining a subsequent scan image using the object scanner with its adjusted configuration and quantifying the quality of the subsequent image) multiple times. The method may carry out the searching step n times, to obtain n subsequent scan images.

The method may then comprise identifying the subsequent scan image whose quality index value indicates that its quality is better than that of the other subsequent scan images. The method may comprise identifying the subsequent scan image whose quality index is the highest amongst the succession of different subsequent scan images, or identifying the subsequent scan image whose quality index is the lowest amongst the succession of different subsequent scan images.

While carrying out the searching step at least once or repeatedly, two (or more) scan images of the succession of subsequent scan images may have the same quality index value. If the quality index values of two scan images are the same, or substantially the same, other parameters (or properties) of those scan images may be used in order to determine which image has the better quality.

For example, one such parameter may be a noise index. The noise index may be defined as the number of, or product of the number of, intensity fluctuations above a threshold in a given region of the image. The given region of the image may correspond to the first region of the scan image, the second region of the scan image, both regions, or a different region. The given region may be a line corresponding to the first region or the second region. Referring to Figure 2, the noise index may be the number of, or product of the number of, grey values of pixels along line 210 that exceed a predetermined threshold. The predetermined threshold may be a predetermined grey value, or range of grey values, and may be set at any value within the range of grey values of the scan image. The threshold may represent a magnitude of the relative prominence or intensity of the grey value of the pixels within a given region of the scan image.

For example, where the two regions are lines across the scan image, the noise index may be the product of the number of intensity fluctuations above a threshold along the line representing the first region, and the number of intensity fluctuations above a threshold along the line representing the second region. Where the number of fluctuations in the first region is 50 and the number of fluctuations in the second region is 10, the noise index may then be 500.

Another such parameter may be scatter index. For 2DCT scan images, the scatter index may be defined as the number of pixels affected by scatter. A pixel is identified as a scatter pixel if the grey value intensity is lower than an object grey value threshold and higher than a background grey value threshold. That is, scatter pixels may be pixels having an intermediary grey value between the grey value of the object and the grey value of the background. Similarly, for 3DCT scan images, the scatter index may be defined as the volume of voxels affected by scatter. A voxel is identified as a scatter voxel if the grey value intensity is lower than the object grey value threshold and higher than the background grey value threshold. That is, scatter voxels may be voxels having an intermediary grey value between the grey value of the object and the grey value of the background.

The thresholds may be defined by the user or calculated statistically based on a defined percentile or percentile range.

Thus, two (or more) scan images with the same quality index value may use a noise value and/or a scatter profile as a tie breaker to select the best quality image.

The above methods may be partly or fully computer-implemented, and thus be considered computer-implemented methods. The methods may be implemented on computing apparatus, or a group of computing apparatuses, configured to receive a measure of a current drawn by the electrical machine and carry out any of the methods described herein.

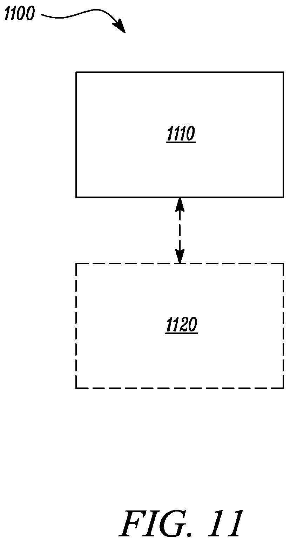

**Figure 11** is a schematic diagram of a system 1100, comprising quality-quantification apparatus 1110 and an object scanner 1120. The object scanner 1120 is indicated as optional to emphasize that in some arrangements the quality-quantification apparatus 1110 may be provided without the object scanner 1120 and operate on scan images provided from (internal or external) storage.

Quality-quantification apparatus 1110 may be configured to implement any of the methods disclosed herein, in some cases in conjunction with the object scanner 1120. Quality-quantification apparatus 1110 may be implemented in software or hardware or in any combination thereof, and may for example be implemented as computing apparatus having one or more processors configured to execute a computer program.

The apparatus 1110 may thus be configured to obtain scan images from the object scanner 1120, and in some cases be configured to adjust the configuration of the object scanner (by adjusting at least one process variable value as mentioned above). The apparatus 1110 may be configured to output the determined QI values to an optional external system (not shown) such that the external system may make decisions based on the QI values.

The object scanner 1120 may be a CT scanner, for example for scanning a turbine blade.

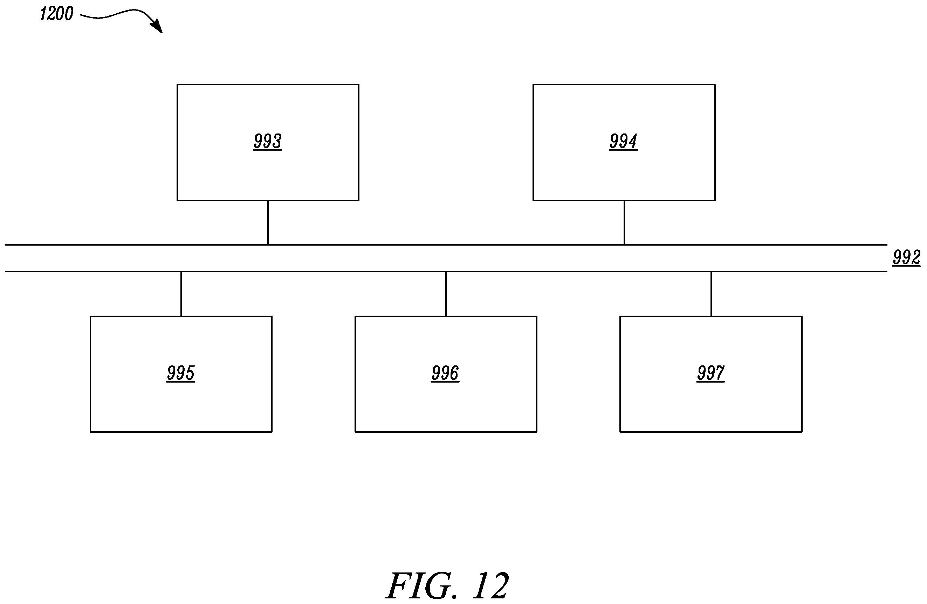

**Figure 12** is a block diagram of a computing device 1200, which may serve to implement the quality-quantification apparatus 1110, and thus which may be used to implement any of the methods of the present disclosure.

The computing device 1200 comprises a processor 993, and memory, 994. Optionally, the computing device also includes a network interface 997 for communication with other computing devices, for example with other computing devices of invention embodiments. For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various methods described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device and may also display a cursor and dialog boxes and screens enabling interaction between a user and the programs and data stored on the computing device. The input mechanisms 996 may enable a user to input data and instructions to the computing device.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

The quality-quantification apparatus 1110 of Figure 11 may thus be a processor 993 (or plurality thereof) executing processing instructions (a program) stored on a memory 994 and exchanging data via a network I/F 997. In particular, the processor 993 may execute processing instructions to receive, via the network I/F, data or signals from the object scanner 1120. Furthermore, the processor 993 may execute processing instructions to store QI values on a connected storage unit and/or to transmit, via the network I/F 997, QI values to an external system for processing. Furthermore, the processor 993 may execute processing instructions to transmit a control signal, via the network I/F 997, to the object scanner 1120.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. A computer-implemented method (500) of quantifying the quality of a scan image of at least a portion of an object against a background, the method comprising:

   deriving (S502) a first measure of contrast and a first measure of sharpness from a first region (610) of the scan image, and a second measure of contrast and a second measure of sharpness from a second region (615) of the scan image; and
   determining (S504) a quality index value indicative of the quality of the scan image based on the first measure of contrast, the first measure of sharpness, the second measure of contrast and the second measure of sharpness.

2. The method (500) of claim 1, wherein,

   a larger measure of contrast is indicative of increased contrast, and wherein the quality index value is determined to be proportional to a product of the first and second measures of contrast; or
   a larger measure of contrast is indicative of decreased contrast, and wherein the quality index value is determined to be inversely proportional to a product of the first and second measures of contrast.

3. The method (500) of claim 1 or 2, wherein each measure of contrast is dependent on, for the region concerned, a ratio of a value of a pixel or voxel representative of the object and a value of a pixel or voxel representative of the background.

4. The method (500) of any of the preceding claims, wherein,

   a larger measure of sharpness is indicative of increased sharpness, and wherein the quality index value is determined to be proportional to a product of the first and second measures of sharpness; or
   a larger measure of sharpness is indicative of decreased sharpness, and wherein the quality index value is determined to be inversely proportional to a product of the first and second measures of sharpness.

5. The method (500) of any of the preceding claims, wherein each measure of sharpness is dependent on, for the region concerned, the width of an edge transition zone representing an edge where the scan image transitions between the object and the background, and/or a gradient of pixel or voxel values in the edge transition zone.

6. The method (500) of any of the preceding claims, wherein:

the first region (610) of the scan image has relatively high contrast and the second region (615) of the scan image has relatively low contrast, optionally wherein the relatively high contrast is higher than a given high contrast threshold and the relatively low contrast is lower than a given low contrast threshold; and/or the first region (610) of the scan image has the highest contrast and the second region (615) of the scan image has the lowest contrast of candidate regions of the scan image.

7. The method (500) of any of the preceding claims, wherein each of said regions is a line or cluster of pixels or voxels which includes at least a part of the object and at least a part of the background.

8. The method (500) of any of the preceding claims, comprising identifying the first (610) and second (615) regions based on user selection and/or based on mathematical analysis of the scan image.

9. The method (500) of any of the preceding claims, wherein the object is of a predetermined type, comprising first and second component sections, and wherein the first region (610) of the scan image is selected to align with at least a part of the first section and wherein the second region (615) of the scan image is selected to align with at least a part of the second section.

10. A computer-implemented method (1000) of assessing the quality of a plurality of scan images, each scan image being of at least a portion of an object against a background, the method comprising:

quantifying (S1002) the quality of each scan image using the method of any of the preceding claims; and assessing (S1004) the quality of the scan images based on their quality index values, optionally wherein the method comprises identifying (S1006), based on the quality index values, one of the scan images whose quality meets a given quality criterion, optionally wherein the quality criterion is that the identified scan image has the best quality of the scan images or has a quality above a given threshold value.

11. The method (1000) of claim 10, comprising, based on the quality index values, identifying a trend or change in scan image quality across the scan images, optionally wherein the plurality of scan images is a series of scan images and the trend or change is identified across the series of scan images.

12. The method (1000) of claim 10 or 11, comprising:

obtaining the scan images using an object scanner configured with one or more process variable values, with at least one process variable value different for each scan image; or obtaining the scan images using an object scanner configured with the same process variable values for each scan image.

13. A computer-implemented method (1020) of obtaining scan images to meet a given quality criterion, each scan image being of at least a portion of an object against a background, the method comprising:

obtaining (S1022) a first scan image using an object scanner configured with one or more process variable values, and quantifying the quality of the first scan image using the method of any of claims 1 to 9; and in a searching step, adjusting (S1024) the configuration of the object scanner by adjusting at least one process variable value, obtaining a subsequent scan image using the object scanner with its adjusted configuration, and quantifying the quality of that subsequent scan image using the method of any of claims 1 to 9, wherein the method comprises carrying out the searching step at least once or repeatedly at least until the quality index values of the obtained scan images indicate that the given quality criterion is met (S1026).

14. The method (1020) of claim 13, wherein the given quality criterion is defined such that it is met when:

the quality index values of the obtained scan images indicate that at least one of the obtained scan images has a quality above a given threshold value; and/or the quality index values of the obtained scan images indicate that the one of the obtained scan images which has the highest quality has a quality which is a given threshold amount better than that of the scan image with the next best quality; and/or

the quality index values of the obtained scan images indicate that the quality of the subsequent scan image of that searching step is better than that of the first scan image or of each other scan image.

15. The method (1000, 1020) of claim any of claims 12 to 14, wherein the method comprises:

adjusting the configuration of the object scanner by referring to a database of image scans and associated process variable values and quality index values, or by referring to a database of process variable values and associated quality index values; and/or

adjusting the configuration of the object scanner based on the quality index value of at least one obtained scan image and/or based on the quality index value of each obtained scan image.

16. The method (500, 1000, 1020) of any of the preceding claims, wherein:

each scan image is a CT scan image, a 3DCT scan image, or a 2DCT scan image derived from a 3DCT scan image; and/or

each scan image is obtained using an object scanner, and/or said object scanner is a CT scanner; and/or

each scan image is an image of a cross-section of at least a portion of the object; and/or

the object has at least one internal or exposed cavity; and/or

the object is a turbine blade; and/or

each quality index value is determined based on a mathematical combination of, or by mathematically combining, the first measure of contrast, the first measure of sharpness, the second measure of contrast and the second measure of sharpness, optionally by multiplication and/or division; and/or

each quality index value is a single and/or integrated value; and/or

each quality index value is a simple number and/or a real number.

17. Quality-quantification apparatus (1110) configured to carry out the method of any of claims 1 to 11.

18. A quality-quantification computer program which, when executed on a computer, causes the computer to carry out the method of any of claims 1 to 11.

19. An image scanning system (1100), comprising:

an object scanner (1120); and

quality-quantification apparatus (1110) configured to communicate with the object scanner, wherein the system is configured to carry out the method of any of claims 1 to 16.

20. An image scanning computer program which, when executed on a computer of an image scanning system, the system comprising an object scanner (1120) and quality-quantification apparatus (1110) configured to communicate with the object scanner (1120), causes the system to carry out the method of any of claims 1 to 16.

**FIG. 1**

**FIG. 2**

EP 4 390 840 A1

400

410

420

Grey value

Horizontal position (pixel)

*FIG. 4*

300

310

330

320

Grey value

Horizontal position (pixel)

*FIG. 3*

500

*S502*

*S504*

*FIG. 5*

FIG. 6

EP 4 390 840 A1

FIG. 7

EP 4 390 840 A1

FIG. 8

EP 4 390 840 A1

FIG. 9

1000

S1002

S1004

S1006

FIG. 10A

*1020*

S1022

S1024

NO

S1026

YES

END

*FIG. 10B*

*1040*

S1042

S1044

S1046

# FIG. 10C

EP 4 390 840 A1

1100

1110

1120

FIG. 11

*FIG. 12*

EP 4 390 840 A1

Europäisches
Patentamt
European
Patent Office
Office européen
des brevets

# EUROPEAN SEARCH REPORT

Application Number

EP 23 21 4522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 610 783 A1 (OPTOS PLC [GB]) 19 February 2020 (2020-02-19) | 1-4,6-20 | INV. G06T7/00 |
| Y | * the whole document * <br> * figures 1-4 * <br> * paragraphs [0004] – [0014] * <br> * paragraphs [0021] – [0028] * <br> * paragraph [0070] * <br> ----- | 5 | ADD. G06V10/25 |
| Y | US 2009/116713 A1 (YAN MICHELLE XIAO-HONG [US] ET AL) 7 May 2009 (2009-05-07) <br> * the whole document * <br> * claim 1 * <br> * paragraphs [0006] – [0007] * <br> ----- | 5 | |
| A | WO 2022/043289 A1 (HOFFMANN LA ROCHE [CH]; ROCHE DIAGNOSTICS GMBH [DE] ET AL.) 3 March 2022 (2022-03-03) <br> * the whole document * <br> * figure 1AB * <br> ----- | 1-20 | |

TECHNICAL FIELDS
SEARCHED      (IPC)

G06T
G06V

The present search report has been drawn up for all claims

| Place of search | Date of completion of the search | Examiner |
|---|---|---|
| The Hague | 19 April 2024 | Thollot, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 4522

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2024

| Patent document cited in search report | | Publication date | Patent family member(s) | | Publication date |
|---|---|---|---|---|---|
| EP 3610783 | A1 | 19-02-2020 | CN 110889820 A | | 17-03-2020 |
| | | | DK 3610783 T3 | | 05-12-2022 |
| | | | EP 3610783 A1 | | 19-02-2020 |
| | | | ES 2932442 T3 | | 19-01-2023 |
| | | | JP 6838112 B2 | | 03-03-2021 |
| | | | JP 2020047264 A | | 26-03-2020 |
| | | | US 2020058122 A1 | | 20-02-2020 |
| US 2009116713 | A1 | 07-05-2009 | NONE | | |
| WO 2022043289 | A1 | 03-03-2022 | CN 115917593 A | | 04-04-2023 |
| | | | EP 4205072 A1 | | 05-07-2023 |
| | | | JP 2023542619 A | | 11-10-2023 |
| | | | US 2023206416 A1 | | 29-06-2023 |
| | | | WO 2022043289 A1 | | 03-03-2022 |

EPO FORM P0459

For more details about this annex : see Official Journal of the European Patent Office, No. 12/82